# EUROPEAN PATENT SPECIFICATION

(11) **EP 4 710 995 B1**
(45) Date of publication and mention of the grant of the patent: **09.12.2026**
(21) Application number: 25202585.3
(22) Date of filing: 16.09.2025
(51) Int. Cl.: A61N 1/375, A61M 25/00, A61M 25/01

(54) **INTEGRATED BEARING HOUSING**
INTEGRIERTES LAGERGEHÄUSE
LOGEMENT DE PALIER INTÉGRÉ

(30) Priority: 17.09.2024 US 202463695779 P; 15.09.2025 US 202519329244
(43) Date of publication of application: 18.03.2026
(73) Proprietor: Pacesetter, Inc., Sylmar, CA 91342 (US)
(72) Inventor: Weber, Adam, Sylmar, CA 91342 (US)
(74) Representative: Barker Brettell Sweden AB

(56) References cited:
- EP-B1- 3 762 096
- EP-B1- 3 965 881
- US-A1- 20230 405 270
- US-B2- 10 758 271

## Description

### TECHNICAL FIELD

The disclosed embodiments relate generally to biostimulators. More specifically, but not exclusively, the disclosed embodiments relate to delivery systems used to implant biostimulators, such as leadless pacemakers, in a patient.

### BACKGROUND

Cardiac pacing by an artificial pacemaker, known more generally as a "biostimulator," provides electrical stimulation of the heart when the natural pacemaker and/or conduction system of the heart fails to provide synchronized atrial and ventricular contractions at healthy rates and intervals. Such antibradycardial pacing provides relief from symptoms, and even life support, for hundreds of thousands of patients. Cardiac pacing can also provide electrical overdrive stimulation to suppress or convert tachyarrhythmias, again supplying relief from symptoms and preventing or terminating arrhythmias that could lead to sudden cardiac death.

Currently available or conventional pacemakers usually perform cardiac pacing using an electrical pulse generator implanted away from the actual pacing site. The pulse generator is typically placed subcutaneously or sub-muscularly in or near the pectoral region of a patient and is connected to the pacing site with leads that are typically 50-70 centimeters long. The operating parameters of the pulse generator are usually interrogated and modified in one of three ways: by a programming device outside the body; via a loosely-coupled transformer with one inductance inside the body and another outside; or via electromagnetic radiation with one antenna inside the body and another outside. The implanted leads have proximal ends connected to the pulse generator and distal ends with one or more electrodes that are positioned adjacent to the inside or outside wall of a cardiac chamber. The leads have an electrical conductor for connecting the pulse generator to electrodes in the heart. Pacing leads can engage and/or be fixed to an intracardial implant site by an engaging mechanism such as an electrode anchor. For example, the electrode anchor can screw into the myocardium.

Leadless cardiac pacemakers locate all electronic circuitry at the pacing site, thereby eliminating electrical leads and avoiding shortcomings associated with conventional cardiac pacing systems. Leadless cardiac pacemakers can be anchored at a pacing site-for instance, in a right ventricle for single-chamber pacing and in both a right ventricle and a right atrium for dual-chamber pacing-by an anchor. A delivery system can be used to deliver the leadless cardiac pacemakers to the target anatomy and a recovery system, which in some instances can be the same as the delivery system, is used to retrieve a leadless pacemaker.

US 10,758,271 B2 discloses a catheter-based delivery system for delivery and retrieval of a leadless pacemaker. The system includes features to facilitate improved manipulation of the catheter and improved capture and docking functionality of leadless pacemakers. Such functionality includes mechanisms directed to deflecting and locking a deflectable catheter, maintain tension on a retrieval feature, protection from anti-rotation, and improved docking cap and driver gear assemblies.

EP 3 762 096 B1 discloses a leadless biostimulator including an attachment feature to facilitate precise manipulation during delivery or retrieval is described. The attachment feature can be monolithically formed from a rigid material, and includes a base, a button, and a stem interconnecting the base to the button. The stem is a single post having a transverse profile extending around a central axis. The transverse profile can be annular and can surround the central axis. The leadless biostimulator includes a battery assembly having a cell can that includes an end boss. A tether recess in the end boss is axially aligned with a face port in the button to receive tethers of a delivery or retrieval system through an inner lumen of the stem. The attachment feature can be mounted on and welded to the cell can at a thickened transition region around the end boss.

EP 3 965 881 B1 discloses a tether head assembly of a delivery system includes an inner retainer and an outer retainer that defines an aperture A tether head assembly of a delivery system includes an inner retainer and an outer retainer that defines an aperture comprising a receptacle configured to receive an attachment member of a medical device, a passageway, and a groove. The inner retainer is movable within the groove between a second position in which the passageway is dimensioned to receive the attachment member and a first position in which the passageway is dimensioned to prevent passage of the attachment member. A tether handle assembly defines a channel, a force transmitter within the channel, a slidable member partially received within a first end of the channel and a button partially received within a second end of the channel.

US 2023/405270 A1 discloses a catheter shaft including a proximal portion with a first polymer having a first stiffness and a first portion of a tubular braid component extending within the first polymer, an intermediate portion with a second polymer having a second stiffness and a second portion of the tubular braid component extending within the second polymer, and a distal portion with a third polymer having a third stiffness. The third portion of the tubular brain component extending within the third polymer. The catheter shaft also includes a hypotube extending withing the third polymer. The hypotube is configured for elastic deformation and includes a sidewall cut with a pattern. Along an overlap segment of the distal portion the third portion of the tubular braid component and the hypotube overlap each other. The outer shaft is configured for improved torque transfer.

### SUMMARY

The invention is defined in the appended claims. Aspects, embodiments and examples disclosed herein which do not fall within the scope of the appended claims do not form part of the invention, and are merely provided for illustrative purposes.

In an aspect, a bearing assembly is adapted to be coupled to a catheter. The bearing assembly includes a housing coupled to the catheter. The housing includes a central opening and an outer bearing race formed in the housing around the central opening. A docking cap stem is positioned in the central opening, is rotatable relative to the housing, and the docking cap stem is adapted to be coupled to a torque shaft of the catheter and forms an inner bearing race, so that the outer bearing race and the inner bearing race together form a bearing race. Several ball bearings are positioned in, and contained by, the bearing race, and a docking cap is coupled to the docking cap stem.

In another aspect, a system for delivering a biostimulator includes a catheter having a proximal end, a distal end, a lumen extending between the proximal end and the distal end, and a torque shaft positioned in the lumen. A bearing assembly is coupled to the catheter. The bearing assembly includes a housing attached to the catheter. The housing includes a central opening and an outer bearing race formed in the housing around the central opening. A docking cap stem is positioned in the central opening, is rotatable relative to the housing, and is adapted to be coupled to a torque shaft of the catheter and forms an inner bearing race, so that the outer bearing race and the inner bearing race together form a bearing race. Several ball bearings are positioned in, and contained by, the bearing race, and a docking cap is coupled to the docking cap stem.

### BRIEF DESCRIPTION OF THE DRAWINGS

Non-limiting and non-exhaustive embodiments of the present invention are described with reference to the following figures, in which like reference numerals refer to like parts throughout the various views unless otherwise specified.
Fig. 1 is a diagrammatic cross section of a human heart illustrating an embodiment of implantation of a biostimulator in a target anatomy.
Figs. 2A-2B are perspective views of an embodiment of a biostimulator system including a biostimulator and a biostimulator transport system catheter.
Figs. 3A-3B are sectional and side views, respectively, of an embodiment of an integrated bearing assembly.
Figs. 4A-4B are sectional and side views, respectively, of another embodiment of an integrated bearing assembly.
Figs. 5A-5B are sectional and side views, respectively, of another embodiment of an integrated bearing assembly.
Figs. 6A-6B are sectional and side views, respectively, of another embodiment of an integrated bearing assembly.

### DETAILED DESCRIPTION

As used in this application, directional terms such as "left," "right," "front," "rear," "upper," lower," "top," "bottom," "side," "lateral," "longitudinal," etc., refer to the orientations of embodiments as they are presented in the drawings, but any directional term should not be interpreted to imply or require a particular orientation of the described embodiments when in actual use. The use of relative terms throughout the description can denote a relative position or direction. For example, "distal" can indicate a first direction along a longitudinal axis of a biostimulator transport system and "proximal" can indicate a second direction opposite to the first direction. Such terms are provided to establish relative frames of reference, but are not intended to limit the use or orientation of the disclosed devices to a specific configuration described in the various embodiments below.

Delivery of a leadless pacemaker (LP) requires applying a torque to the LP to fix its helix into the target tissue. This rotation to supply torque to the LP must be isolated from the catheter's deflection, so that the torque and linear motion do not affect each other-i.e., torque does not affect linear motion and linear motion does not affect torque. Isolating the rotation and torque supply ensures the catheter can be deflected to put the LP in the correct position and can maintain the correct position while the LP is rotated to fixate the helix in the target location. Existing assemblies at the distal end of a catheter use a manufacturer's existing bearing that is captured in place to isolate torque from the catheter. But there is limited space available within the right ventricle and atrium, meaning that a reduction in rigid length-i.e., the length of the rigid components at the distal end of the catheter-is desirable to promote better catheter steerability.

This application discloses embodiments of distal bearing assemblies that reduce rigid length by integrating the bearing surface into the distal assembly. Reducing the distal rigid length improves the catheter's range of deflection and ability to be positioned. An outer housing and docking cap contain inner and outer races for ball bearings, and a pull wire can be directly retained in the outer housing, so that it serves as an externalized pull-ring to deflect the catheter. The docking cap remains coaxial with the deflection and the ball bearings isolate its torque and thrust loading. This method of integrated bearing may be used across any concept requiring torque to implant the LP.

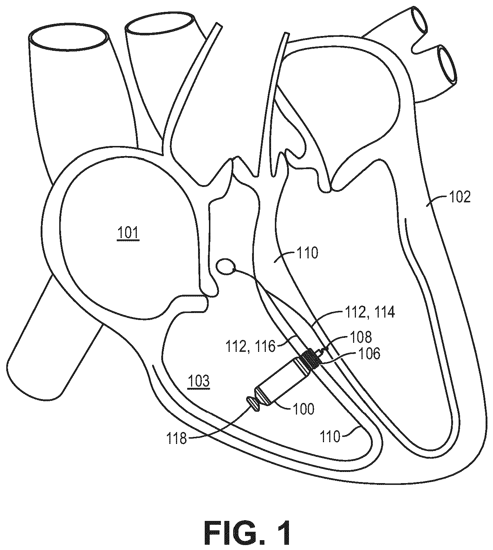

Fig. 1 illustrates an embodiment of implantation of a biostimulator in a target anatomy of a human heart. A leadless biostimulator system, e.g., a cardiac pacing system, includes one or more biostimulators 100 that can be implanted in a patient heart 102 and can be leadless (e.g., it can be a leadless pacemaker (LP)). Each biostimulator can be placed in a cardiac chamber, such as a right atrium 101 and/or right ventricle 103, or attached to an inside or outside of the cardiac chamber. For example, biostimulator 100 can be attached to a septum 110 of heart 102. More particularly, biostimulator 100 can be delivered to the septum 110, and one or more elements, such as a fixation element 106 and/or a pacing element 108, can pierce septal wall 110 to engage and anchor the biostimulator 100 to the target anatomy, e.g., a bundle branch 112 in the septal wall 110. In a particular embodiment, biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

When biostimulator 100 is delivered to and screwed into the target tissue, e.g., an atrial or ventricular wall, or a septum 110 of the heart 102, pacing element 108 and/or fixation element 106 can be positioned for pacing. For example, in the case of deep septal pacing at respective bundle branches 112 in septum 110, an active electrode of pacing element 108 can be positioned at a first target anatomy in the septal wall 110, e.g., a left bundle branch 114. Similarly, the fixation element 106 can be positioned at a second target anatomy in the septal wall, e.g., a right bundle branch 116. Optionally, one of the elements may be at a bundle branch and the other element may not be at a bundle branch.

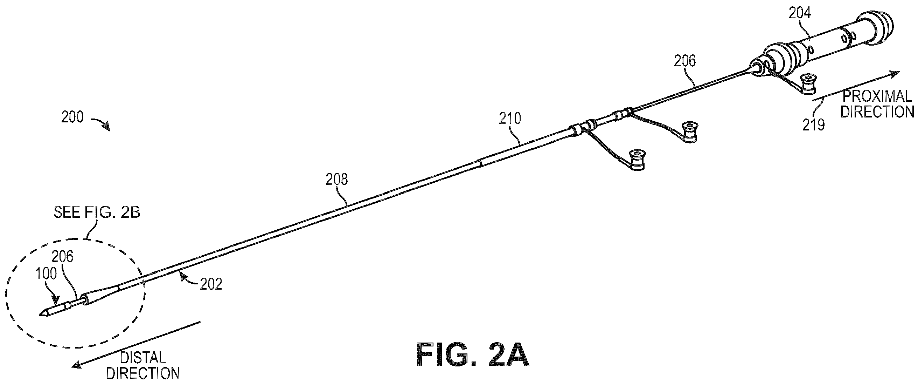

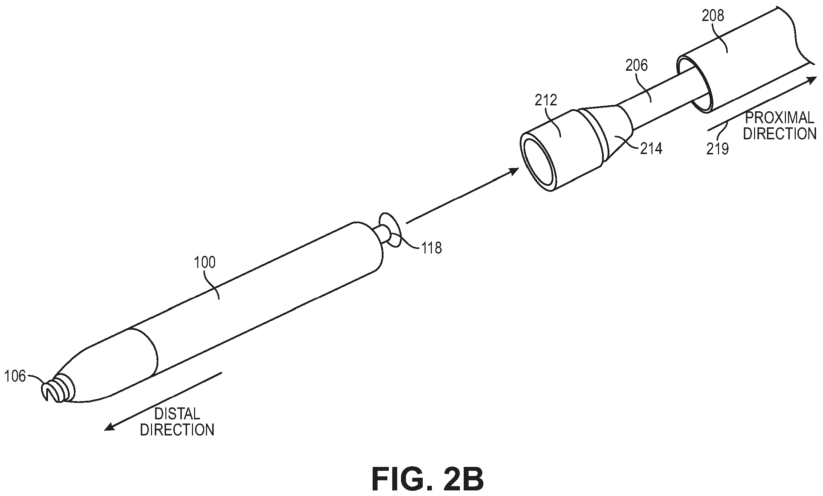

Figs. 2A-2B illustrate an embodiment of a biostimulator system 200. Fig. 2A is perspective view of the biostimulator system, and Fig. 2B is a perspective view of the distal end of the system. Biostimulator system 200 can include a biostimulator 100, e.g., a leadless pacemaker or other leadless biostimulator. System 200 can also include delivery or retrieval systems, which can be catheter-based systems used to carry biostimulator 100 intravenously to or from a patient anatomy. For example, a biostimulator transport system 202 can be used to deliver biostimulator 100 to, or retrieve the biostimulator from, a patient. Biostimulator 100 can be attached, connected to, or otherwise mounted on biostimulator transport system 202. The biostimulator can be mounted on a distal end of a catheter of the biostimulator transport system 202, which catheter is then used to advance biostimulator 100 intravenously into or out of heart 102.

Biostimulator transport system 202 can include a handle 204 to control movement and operations of the transport system from outside a patient. One or more elongated members extend distally from handle 204. For example, an elongated catheter body 206 can extend distally from handle 204 to a distal end of the biostimulator transport system. In an embodiment, biostimulator 100 is mounted on a distal end of elongated catheter body 206.

Biostimulator transport system 202 can include a protective sleeve 208 to cover biostimulator 100 during delivery and implantation. Protective sleeve 208 can extend over, and be longitudinally movable relative to, elongated catheter body 206. The biostimulator transport system can also include an introducer sheath 210 that can extend over, and be longitudinally movable relative to, the protective sleeve 208. Introducer sheath 210 can cover a distal end of the protective sleeve 208, the elongated catheter body 206, and biostimulator 100, as those components are passed through an access device into the patient anatomy.

Biostimulator transport system 202 can also be configured to include additional or alternate components. More particularly, the biostimulator transport system can be configured to deliver biostimulator 100 to, or retrieve it from, the target anatomy. Delivery and/or retrieval of biostimulator 100 can include retaining the biostimulator 100 on catheter 102 during transport to the target anatomy and rotation of the biostimulator during its implantation at the target anatomy. Accordingly, biostimulator transport system 202 can incorporate features to retain and rotate biostimulator 100, including at least those described below.

Fig. 2B illustrates an embodiment of a distal portion of a biostimulator delivery system and its attachment to a biostimulator. The view is a close-up view of a distal portion of the biostimulator transport system 200 shown in Fig. 2A. The delivery system can include features to engage the leadless pacemaker and to allow screwing the biostimulator 100 into the target tissue.

Similar to the retrieval system, the delivery system can include the docking cap 212 having a key configured to engage attachment feature 118 of the leadless pacemaker and apply torque to screw the fixation element 106 into the target tissue. The delivery system may also include the elongated catheter 206 having a torque shaft on which docking cap 212 and bearing housing 214 are mounted. The protective sheath 208 can be positioned along the outer catheter 206, and can be advanced or retracted to cover or expose the docking cap 212 and leadless pacemaker 100.

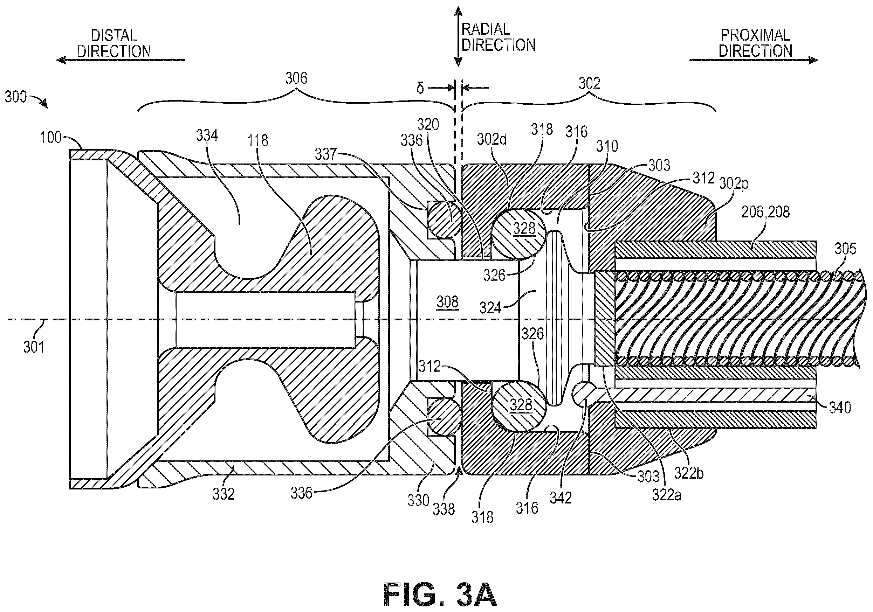

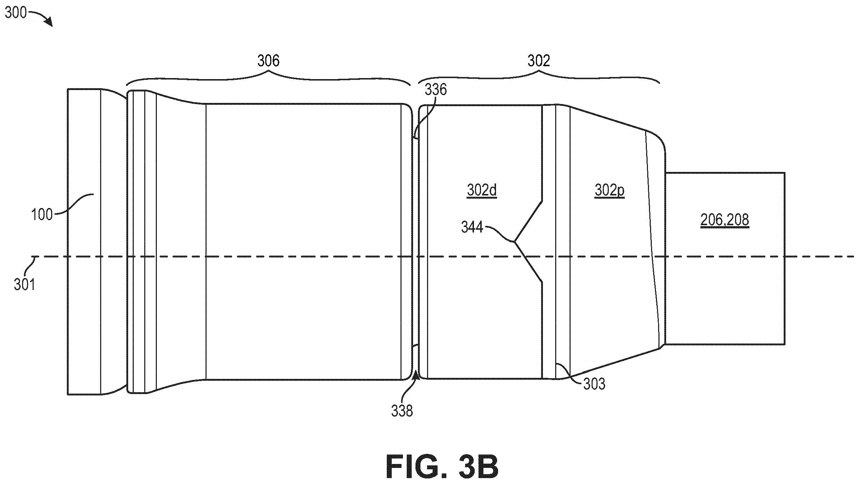

Figs. 3A-3B together illustrate an embodiment of a bearing assembly 300. Fig. 3A is a cross-sectional view of the assembled bearing assembly, and Fig. 3B is an exterior view of the assembled bearing assembly.

Bearing assembly 300 includes three main parts arranged along a longitudinal axis 301: a housing 302 coupled to the distal end of a catheter 206, a docking cap 306 positioned distally from housing 302, and docking cap stem 308 joining the housing and the docking cap. In the illustrated embodiment the three main parts are substantially centered on longitudinal axis 301, but in other embodiments one or more of the main parts need not be so centered.

Housing 302 has a proximal housing part 302p and a distal housing part 302d. When joined together along seam 303, proximal housing part 302p and distal housing part 302d form an internal lumen 310 that is bounded by a proximal wall 312 of distal housing part 302d, a distal wall 314 of proximal housing part 302p, and a radial wall 316 of distal housing part 302d. An outer bearing race 318 is formed substantially where radial wall 316 joins distal wall 312. Both proximal housing part 302p and distal housing part 302d include a central opening centered on longitudinal axis 301. In distal housing part 302d, central opening 320 is used to receive a distal part of docking cap stem 308, while in proximal housing part 302p the central opening 322 is used to receive a proximal part of docking cap stem 308 and a distal end of catheter 206. Central opening 322 includes two parts with different diameters: a first part 322a with a smaller diameter to receive the docking cap stem and a second part 322b with a larger diameter than first part 322a to receive catheter 206 and elements within the catheter such as torque shaft 305.

Docking cap stem 308 has a proximal end and a distal end and is used to couple docking cap 306 to torque shaft 305 and housing 302. In the illustrated embodiment, the distal end of the docking cap stem is fixed to the docking cap and the proximal end of the docking cap stem is fixed to torque shaft 305. The docking cap stem can rotate relative to the housing, so that torque applied to the torque shaft is transmitted, through the docking cap stem, to the docking cap but not to the housing. When positioned in central openings 320 and 322a, docking cap stem 308 extends from a position distal to the housing, through central opening 320, into internal lumen 310, and into central opening 322a. Once positioned in the housing, the distal end of the docking stem projects distally outside the housing so that it can be joined to docking cap 306 and its proximal end is attached to torque shaft 305. The part of the docking cap stem that is within internal lumen 310 includes a flared portion 324 (i.e., a portion with a larger radius than the rest of the stem). The flared portion forms a curved surface 326 that functions as an inner bearing race. Inner bearing race 326 and outer bearing race 318 together form a complete bearing race to retain ball bearings 328.

Several ball bearings 328 are positioned in internal lumen 310, where they are held in the bearing race formed by inner bearing race 326 and outer bearing race 318. Docking cap stem 308 rotates relative to housing 302, and the ball bearings facilitate its rotation. Bearings 328 can be any of several types of bearing. In an embodiment bearing 328 can be a plain ball bearings but other embodiments can use other types of bearings.

Docking cap 306 is adapted to receive an end of a biostimulator 100 such as a leadless pacemaker (LP). The docking cap includes a base 330 and a distal cup portion 332 that projects distally from the base. Distal cup 332 forms a docking cavity 334, which can be a generally cylindrically-shaped void within the distal cup. The docking cavity 334 can be sized and configured to receive the attachment feature of biostimulator 100. In some embodiments, features such as a torque key (not shown) can be located within the docking cavity 334 to engage attachment feature 118 and transmit torque to biostimulator 100. Base 330 is coupled to the distal end of docking cap stem 308, so that torque is transmitted from torque shaft 305 to docking cap 306 through docking cap stem 308.

A seal 336 is positioned in a groove 337 on base 330 to seal the gap 338 between the proximal-facing side of base 330 and the distal-facing side of distal housing part 302d. Seal 336 also provides a minimal thrust loading to ensure the ball bearings do not lose their orientation and, in an embodiment, the docking cap stem contains a locating feature to control orientation and optimal positioning of the O-ring. In an embodiment, the width of gap 338 can be set so that the viscosity or other physical properties of bodily fluids encountered by bearing assembly 300 when in use-usually, but not necessarily, blood-will reduce or prevent infiltration of the bodily fluids into internal lumen 310 where it could impair the smooth operation of bearings 328. Seal 336 and gap 338, acting on their own or in combination, reduce or prevent this infiltration. In an embodiment seal 336 can be an O-ring, but other embodiments can use other types of seals. Among other things, reducing or eliminating infiltration of blood into internal lumen 310 reduces or eliminates the need to constantly flush the internal lumen with an anti-coagulant solution such as heparinized saline, thus also reducing or eliminating the need for flushing ports in housing 302 and the need for fluid lines to deliver the anti-coagulant preparation to the flushing ports.

A pull wire 340 runs through a lumen of catheter 304 and into internal lumen 310 through a pull-wire hole in proximal housing part 302p, so that the distal end of the pull wire is within internal lumen 310 and the pull wire extends proximally from the distal end within catheter 304. The pull wire is used to create radial deflection (i.e., deflection normal to longitudinal axis 301) of bearing assembly 300. The pull wire includes a retainer 342 formed on its distal end to create an interference that prevents the distal end from being pulled out of the internal lumen when a proximal force is applied to the pull wire. In the illustrated embodiment retainer 342 is a sphere or spheroid formed on the distal end of the pull wire, but in other embodiments the retainer can be a different structure than shown, e.g., a crimp band.

To assemble bearing assembly 300, bearings 328 are first positioned in internal lumen 310 and pull wire 340 is inserted before assembly of the housing. The distal end of docking cap stem 308 is then inserted in a distal direction into hole 320, so that surfaces 318 and 326 form the bearing race within which bearings 328 are held. The proximal end of docking cap stem 318 is then inserted into hole 322a, and proximal housing part 302p and distal housing part 302d are joined together along seam 303, for instance by welding in one embodiment, to complete housing 302. A pointed feature 344 (see Fig. 3B) helps align the proximal and distal housing parts of the housing so that they are joined in the correct orientation. The pointed feature 344 can be a projection from one housing part that is received by a recess of the other housing part to cause a rotational interference. Accordingly, the pointed feature 344 can resist relative rotation between the parts when engaged. Seal 336 is then positioned in groove 337 on base 330, and then docking cap 306 is joined to the distal end of docking cap stem 308, for instance by welding in one embodiment.

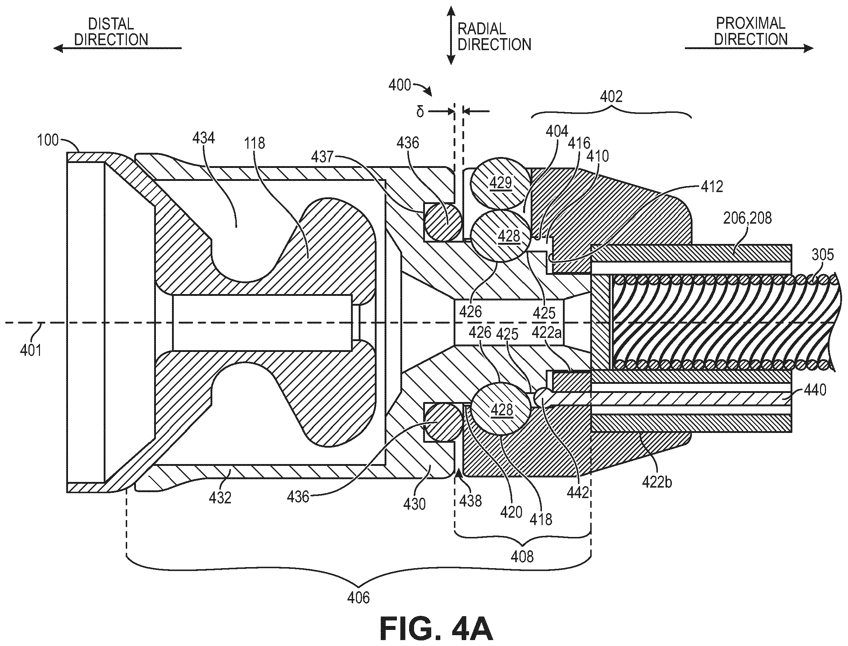

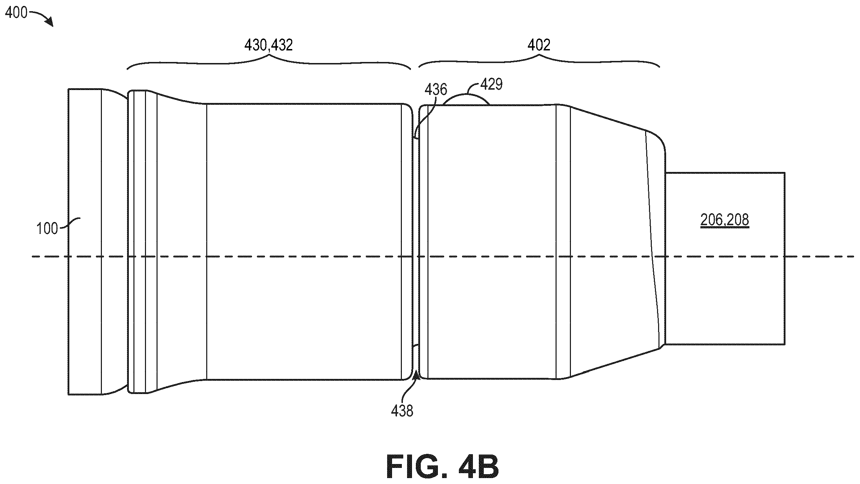

Figs. 4A-4B together illustrate an embodiment of an integrated bearing assembly 400. Fig. 4A is a cross-sectional view of the assembled bearing assembly, and Fig. 4B is an exterior view of the assembled bearing assembly.

Bearing assembly 400 includes two main parts arranged along a longitudinal axis 401: a housing 402 coupled to the distal end of a catheter 206 and a docking cap 406, part of which is positioned distally from housing 402 and part of which is within housing 402. A principal difference between bearing assembly 400 and bearing assembly 300 is that bearing assembly 400 replaces the docking cap stem with a proximal projection 408 of the docking cap. Put differently, in bearing assembly 400 the docking cap stem is integrated into the docking cap by the docking cap projection, so that the docking cap stem is not a separate part. More particularly, the docking cap and the docking cap stem can be a single piece.

Housing 402 is a single solid piece with an internal lumen 410 that is bounded by a proximal wall 412 and a radial wall 416. The housing includes a central opening centered on longitudinal axis 401. At the distal end of the housing, central opening 420 receives docking cap projection 408; at the proximal end of the housing, the central opening includes two parts with different diameters: a first part 422a with a smaller diameter to receive the docking cap projection and a second part 422b with a larger diameter than first part 422a to receive catheter 206 and elements within the catheter such as torque shaft 305. An outer bearing race 418 is formed in radial wall 416, and an access hole 404 is formed in the housing, by drilling In an embodiment, and extends through the thickness of the housing from radial wall 416 to the exterior of the housing. Access hole 404 can be used to insert bearings into the bearing race and, once the bearings are in place, to retain them there.

Docking cap 406 is adapted to receive an end of a biostimulator 100 such as a leadless pacemaker (LP). The docking cap includes a base 430, a distal cup portion 432 that projects distally from the base, and a docking cap projection 408 that projects proximally from the base. Distal cup 432 forms a docking cavity 434, which can be a generally cylindrically-shaped void within the distal cup. The docking cavity 434 can be sized and configured to receive attachment feature 118 of the biostimulator 100. In some embodiments, features such as a torque key can be located within the docking cavity 434 to engage attachment feature 118 and transmit torque from docking cap 406 to biostimulator 100.

Docking cap projection 408 has a proximal end, a distal end, and an outer surface 425. Docking cap projection 408 integrates the docking cap stem into the docking cap, so that they need not be separate pieces as in bearing assembly 300. A proximal end of the docking cap projection is inserted into housing 402 and is used to couple docking cap 406 to torque shaft 305. In the illustrated embodiment, the distal end of the docking cap projection is integrated with the docking cap base 430. The entire docking cap-distal cup 432, base 430, and docking cap projection 408-can rotate relative to housing 402, so that torque applied to torque shaft 305 is transmitted, through docking cap projection 408, to docking cap 406 but not to housing 402.

The docking cap projection is inserted into central openings 420 and 422a and it projects distally from the housing so that its distal end integrates into docking cap base 430. When positioned in central openings 420 and 422a, docking cap projection 408 extends from a position distal to the housing, through central opening 420, into internal lumen 410, and into central opening 422a. The part of the docking cap projection that is within internal lumen 410 includes a groove or curved surface 426 formed in outer surface 425, by machining In an embodiment, that functions as an inner bearing race. Inner bearing race 426 and outer bearing race 418 together form a complete bearing race to retain bearings 428.

Several ball bearings 428 are positioned in the bearing race formed by inner bearing race 426 and outer bearing race 418. As docking cap projection 408 rotates relative to housing 402, the ball bearings facilitate its rotation. Bearings 428 can be any of several types of bearings that permit radial rotation of the docking cap projection 408 relative to housing 402. In an embodiment bearing 428 can be plain ball bearings, but other embodiments can use other types of bearings.

A seal 436 is positioned in a groove 437 on base 430, so that the seal seals the gap 438 between the proximal-facing side of base 430 and the distal-facing side of housing 402. In an embodiment, the width of gap 438 is set so that the viscosity or other physical properties of bodily fluids encountered by bearing assembly 400 when in use-usually, but not necessarily, blood-will reduce or prevent infiltration of the bodily fluids into internal lumen 410 where it could impair the smooth operation of bearings 428. Seal 436 and gap 438, acting on their own or in combination, reduce or prevent this infiltration. In an embodiment seal 436 can be an O-ring, but other embodiments can use other types of seals. Reducing or eliminating infiltration of blood into internal lumen 410 reduces or eliminates the need to flush the internal lumen with an anti-coagulant solution such as heparinized saline, thus also reducing or eliminating the need for flushing ports in housing 402 and the need for fluid lines to deliver the anti-coagulant preparation to the flushing ports.

A pull wire 440 runs through a lumen of catheter 206 and into internal lumen 410 through a pull-wire hole in housing 402, so that the distal end of the pull wire is within internal lumen 410 and the proximal portion extends through the catheter 206. The pull wire is used to create radial movement (i.e., movement normal to longitudinal axis 401) of bearing assembly 400. The pull wire includes a retainer 442 formed on its distal end to prevent the distal end from being pulled out of the internal lumen when a proximal force is applied to the pull wire. In the illustrated embodiment retainer 442 is a sphere or spheroid formed on the distal end of the pull wire, but in other embodiments the retainer can be a different structure than shown.

To assemble bearing assembly 400, pull wire 440 is first inserted into the pull-wire hole. Seal 436 is then positioned in groove 437 on base 430, and the distal end of docking cap projection 408 is inserted into central opening 420 until the proximal end of the docking cap projection is in central opening 422a, and the proximal end of the docking cap projection is coupled to torque shaft 305. Ball bearings 428 are then loaded through external access hole 404. When the ball bearings have been loaded, a final bearing or pin 429 will be placed in the access hole and welded into place as a stopper to ensure a seal and correct function.

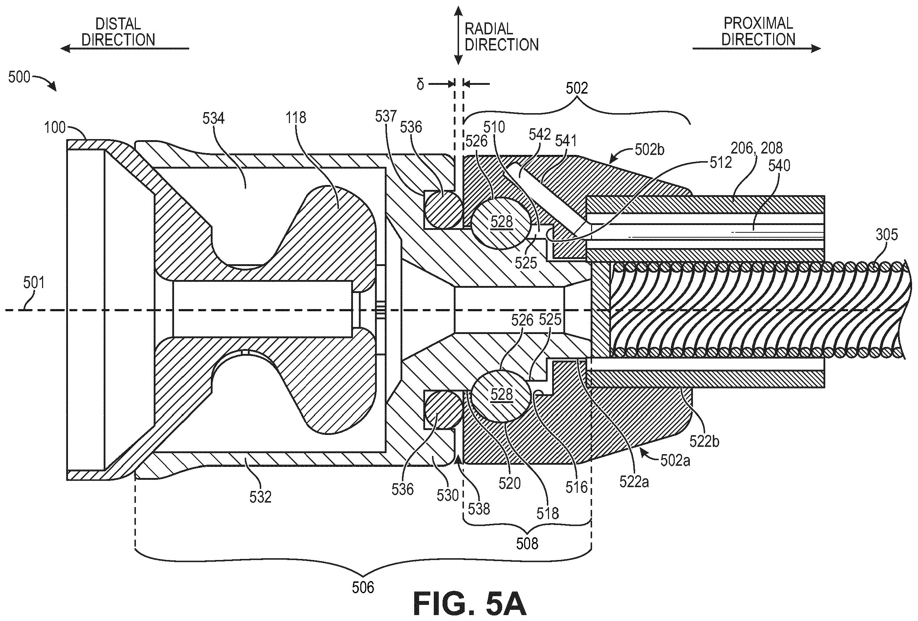

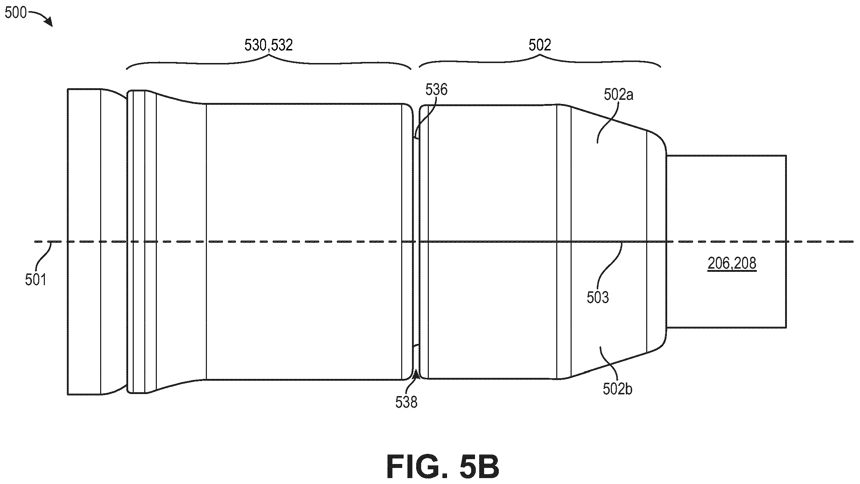

Figs. 5A-5B together illustrate an embodiment of an integrated bearing assembly 500. Fig. 5A is a cross-sectional view of the assembled bearing assembly, and Fig. 5B is an exterior view of the assembled bearing assembly.

Bearing assembly 500 includes two main parts arranged along a longitudinal axis 501: a housing 502 coupled to the distal end of a catheter 206 and a docking cap 506, part of which is positioned distally from housing 502 and part of which is within housing 502. As in bearing assembly 400, in bearing assembly 500 the docking cap stem is integrated into the docking cap, so that the docking cap stem is not a separate part.

Housing 502 has a first part 502a and a second part 502b that are radially separate from each other. When joined together along longitudinal seam 503 (see Fig. 5B) to form housing 502, first part 502a and second part 502b form an internal lumen 510 that is bounded by a proximal wall 512 and a radial wall 516. The housing includes a central opening centered on longitudinal axis 501. At the distal end of the housing, central opening 520 receives docking cap projection 508. At the proximal end of the housing, the central opening includes two parts with different diameters: a first part 522a with a smaller diameter to receive the docking cap projection and a second part 522b with a larger diameter than first part 522a to receive catheter 206 and elements within the catheter such as torque shaft 305. An outer bearing race 518 is formed in radial wall 516. An access hole 504 is formed in the housing and extends through the thickness of the housing from radial wall 516 to the exterior of the housing. Access hole 504 can be used to insert bearings into the bearing race and, once the bearings are in place, to retain them there.

Docking cap 506 is adapted to receive an end of a biostimulator 100 such as a leadless pacemaker (LP). The docking cap includes a base 530, a distal cup portion 532 that projects distally from the base, and a docking cap projection 508 that projects proximally from the base. Distal cup 532 forms a docking cavity 534, which can be a generally cylindrically-shaped void within the distal cup. The docking cavity 534 can be sized and configured to receive attachment feature 118 of the biostimulator 100. In some embodiments, features such as a torque key (not shown) can be located within the docking cavity 534 to engage attachment feature 118 and transmit torque to biostimulator 100.

Docking cap projection 508 has a proximal end, a distal end, and an outer surface 525. Docking cap projection 508 integrates the docking cap stem into the docking cap, so that they need not be separate parts as in bearing assembly 300. A proximal end of the docking cap projection is inserted into housing 502 and is used to couple docking cap 506 to torque shaft 305. In the illustrated embodiment, the distal end of the docking cap projection is integrated with the docking cap base 530. The entire docking cap-distal cup 532, base 530, and docking cap projection 508-can rotate relative to housing 502, so that torque applied to torque shaft 305 is transmitted, through docking cap projection 508, to docking cap 506 but not to housing 502.

The docking cap projection is inserted into central openings 520 and 522a and it projects distally from the housing so that its distal end integrates into docking cap base 530. When positioned in central openings 520 and 522a, docking cap projection 508 extends from a position distal to the housing, through central opening 520, into internal lumen 510, and into central opening 522a. The part of the docking cap projection that is within internal lumen 510 includes a groove or curved surface 526 formed in outer surface 525, by machining In an embodiment; curved surface 526 functions as an inner bearing race. Inner bearing race 526 and outer bearing race 518 together form a complete bearing race to retain bearings 528.

Several ball bearings 528 are positioned in the bearing race formed by inner bearing race 526 and outer bearing race 518. As docking cap projection 508 rotates relative to housing 502, the ball bearings facilitate its rotation. Bearings 528 can be any of several types of bearings that permit rotation of the docking cap projection 508 relative to housing 502. In an embodiment bearing 528 can be a plain ball bearings, but other embodiments can use other types of bearings.

A seal 536 is positioned in a groove 537 on base 530 to seal the gap 538 between the proximal-facing side of base 530 and the distal-facing side of housing 502. In an embodiment, the width of gap 538 is set so that the viscosity or other physical properties of bodily fluids encountered by bearing assembly 500 when in use-usually, but not necessarily, blood-will reduce or prevent infiltration of the bodily fluids into internal lumen 510 where it could impair the smooth operation of bearings 528. Seal 536 and gap 538, each on their own or in combination, reduce or prevent this infiltration. In an embodiment seal 536 can be an O-ring, but other embodiments can use other types of seals. Reducing or eliminating infiltration of blood into internal lumen 510 reduces or eliminates the need to flush the internal lumen with an anti-coagulant solution such as heparinized saline, thus also reducing or eliminating the need for flushing ports in housing 502 and the need for fluid lines to deliver the anti-coagulant preparation to the flushing ports.

A pull wire 540 runs through a lumen of catheter 206 and a distal extension 542 of the pull wire is positioned and fixed in a channel 541 formed in part 502b of the housing. The channel 541 can have a contour, e.g., a U-shaped channel, an angular channel, an L-shaped channel, a bend, etc., such that a section of the channel extends longitudinally and a section extends laterally. In an embodiment the distal extension 542 is fixed in channel 541 by welding, but other embodiments can use other methods. Because distal extension 542 is fixed in groove 541, it prevents the distal end of pull wire 540 from being pulled out of the housing when a proximal force is applied to the pull wire. The pull wire is used to create radial movement (i.e., movement normal to longitudinal axis 501) of bearing assembly 500.

To assemble bearing assembly 500, distal extension 542 of pull wire 540 is first inserted into groove 541 and fixed in place. Seal 536 is then positioned in groove 537 on base 530. Bearings 528 are loaded in first part 502a of the housing and the distal end of docking cap projection 508 is inserted into central opening 520 until the proximal end of the docking cap projection is in central opening 522a, and the proximal end of the docking cap projection is coupled to torque shaft 305. Once docking cap projection 508 is inserted this way, the curved surfaces 518 and 526 retain the bearings in the first part of the housing. Bearings 528 are then loaded into the second part 502b of the housing, and the second part of the housing is assembled to the first part 502a and the docking cap projection 508. First part 502a and second part 502b are then joined together along longitudinal seam 503 (see Fig. 5B), for instance by welding, to complete housing 502.

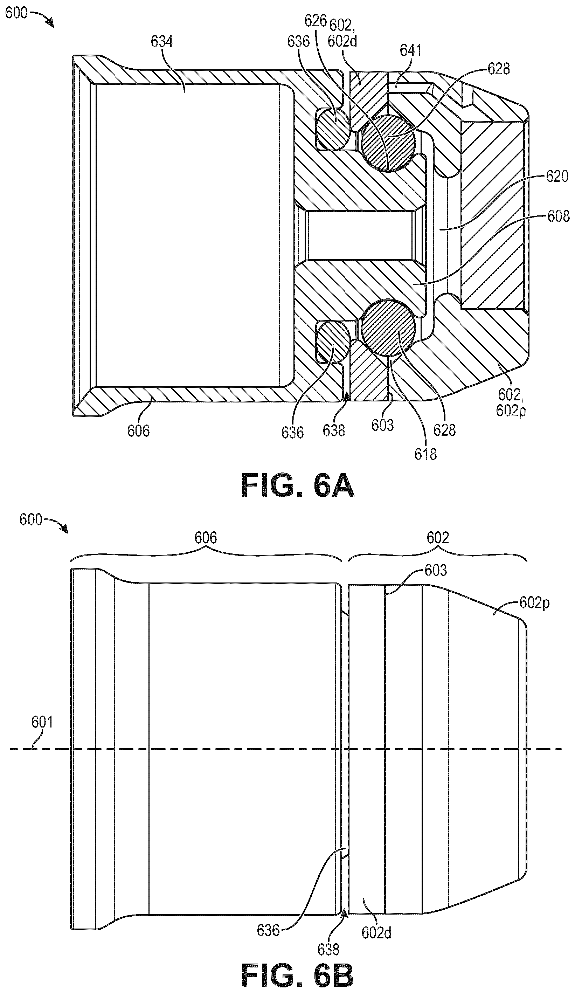

Figs. 6A-6B together illustrate another embodiment of an integrated bearing assembly 600. Fig. 6A is a cross-sectional view of the assembled bearing assembly, and Fig. 6B is an exterior view of the assembled bearing assembly.

Bearing assembly 600 includes two main parts arranged along a longitudinal axis: a housing 602 coupled to the distal end of a catheter (not shown, but including catheter body 206 and protective sleeve 208 described above) and a docking cap 606, part of which is positioned distally from housing 602 and part of which is within housing 602. As in bearing assemblies 400 and 500, in bearing assembly 600 the docking cap stem is integrated into the docking cap, so that the docking cap stem may not be a separate part.

Housing 602 has a proximal housing part 602p and a distal housing part 602d that are separate from each other. When joined together along transverse seam 603 to form housing 602, proximal housing part 602p and distal housing part 602d form an internal lumen that is bounded by internal walls of the housing parts. The housing includes a central opening 620 centered on the longitudinal axis 601. At the distal end of the housing, the central opening receives the docking cap projection. At the proximal end of the housing, the central opening includes portions with different diameters to receive the docking cap projection and catheter elements such as a torque shaft. An outer bearing race 618 is formed in the housing 602. For example, the housing 602 portions, e.g., the distal housing part 602d and the proximal housing part 602p, may include respective angled surfaces that combine to form the race. More particularly, the outer bearing race may include angled surfaces, such as 45-degree surfaces, on each housing part to create the outer bearing surface for the ball bearings to ride within.

Docking cap 606 is adapted to receive an end of a biostimulator such as a leadless pacemaker. The docking cap includes a base and a distal cup portion that projects distally from the base. The distal cup forms a docking cavity 634, which can be a generally cylindrically-shaped void within the distal cup. The docking cavity 634 can be sized and configured to receive the attachment feature 118 of the biostimulator 100. In some embodiments, features such as a torque key can be located within the docking cavity 634 to engage the attachment feature and transmit torque to the biostimulator.

The docking cap 606 includes a docking cap projection 608 that projects proximally from the base and integrates the docking cap stem into the docking cap. A proximal end of the docking cap projection 608 is inserted into housing 602 and is used to couple docking cap 606 to a torque shaft 305. The entire docking cap 606 can rotate relative to housing 602, so that torque applied to the torque shaft 305 is transmitted through the docking cap projection 608 to docking cap 606 but not to housing 602.

The docking cap projection 608 extends into the internal lumen of the housing 602. The part of the docking cap projection 608 that is within the internal lumen includes a U-shaped groove formed in its outer surface that functions as an inner bearing race 626. The U-shaped groove provides a bearing surface for ball bearings 628 to have proper contact and rotation. Inner bearing race 626 and the outer bearing race 618 together form a complete bearing race to retain ball bearings 628.

Several ball bearings 628 are positioned in the bearing race formed by inner bearing race 626 and the outer bearing race 618. As the docking cap projection 608 rotates relative to housing 602, the ball bearings 628 facilitate its rotation. Ball bearings 628 can be any of several types of bearings that permit rotation of the docking cap projection relative to housing 602. In an embodiment ball bearings 628 can be plain ball bearings, but other embodiments can use other types of bearings.

A seal 636 is positioned in a groove on the base of the docking cap 606, so that the seal seals gap 638 between the proximal-facing side of the base and the distal-facing side of housing 602. In an embodiment, the width of gap 638 may be set so that the viscosity or other physical properties of bodily fluids encountered by bearing assembly 600 when in use-usually, but not necessarily, blood-will reduce or prevent infiltration of the bodily fluids into the internal lumen where it could impair the smooth operation of ball bearings 628. Seal 636 and gap 638, acting on their own or in combination, reduce or prevent this infiltration. In an embodiment seal 636 can be an O-ring, but other embodiments can use other types of seals. In some cases, device sealing, e.g., sealing a proximal handle, may be sufficient to keep blood from occluding the bearing, and the O-ring may not be required based on testing results. Reducing or eliminating infiltration of blood into the internal lumen reduces or eliminates the need to flush the internal lumen with an anti-coagulant solution such as heparinized saline, thus also reducing or eliminating the need for flushing ports in housing 602 and the need for fluid lines to deliver the anti-coagulant preparation to the flushing ports.

A pull wire 540 (not shown in FIG. 6A but similar to the pull wire shown in FIG. 5A) runs through a lumen of the catheter and a distal extension of the pull wire is positioned and fixed in a pull wire groove 641 formed in one part of the housing. The pull wire groove 641 may be angled and/or include a bend to accommodate a bent configuration of the pull wire. More particularly, the pull wire can be bent to fit in the groove, similar to the embodiments described above. In an embodiment the distal extension of the pull wire is fixed in the groove by welding, but other embodiments can use other methods, such as mechanical interferences. Because the distal extension is fixed in pull wire groove 641, it prevents the distal end of the pull wire 540 from being pulled out of the housing when a proximal force is applied to the pull wire. The pull wire 540 is used to create radial movement of bearing assembly 600, as described above.

To assemble bearing assembly 600, the distal extension of the pull wire 540 is first inserted into pull wire groove 641 and fixed in place. Seal 636 is positioned in its groove on the base of the docking cap 606. The distal housing part 602d can be loaded over the docking cap stem, against the seal 636. Ball bearings 628 are loaded into the U-shaped groove of the docking cap stem. The proximal housing part 602p can be inserted over the ball bearings 628 and against the distal housing part 602d. The distal and proximal housing parts can be joined, e.g., by welding, to join the parts together along transverse seam 603. The proximal end of the docking cap projection can be coupled to the torque shaft and/or the catheter shaft can be inserted and bonded to the proximal housing part 602p.

## Claims

1. A bearing assembly (300, 400, 500, 600), comprising:
a housing (302, 402, 502, 602) adapted to be coupled to a catheter (206, 304), the housing (302, 402, 502, 602) including a central opening (320, 420, 520, 620) and an outer bearing race (318, 418, 518, 618) formed in the housing (302, 402, 502, 602) around the central opening (320, 420, 520, 620);
a docking cap stem (308, 408, 508, 608) positioned in the central opening (320, 420, 520, 620) and rotatable relative to the housing (302, 402, 502, 602), the docking cap stem (308, 408, 508, 608) adapted to be coupled to a torque shaft (305) of the catheter (206, 304);
a plurality of ball bearings (328, 428, 528, 628); and
a docking cap (306, 406, 506, 606) coupled to the docking cap stem (308, 408, 508, 608), **characterized in that**
the docking cap stem (308, 408, 508, 608) forms an inner bearing race (316, 426, 526, 626) and wherein the outer bearing race (318, 418, 518, 618) and the inner bearing race (316, 426, 526, 626) together form a bearing race; and
the plurality of ball bearings (328, 428, 528, 628) are positioned in, and contained by, the bearing race.

2. The bearing assembly of claim 1, further comprising a seal (336, 436, 536, 636) positioned between the docking cap (306, 406, 506, 606) and the housing (302, 402, 502, 602).

3. The bearing assembly of claim 2, wherein the seal (336, 436, 536, 636) is an O-ring.

4. The bearing assembly of any one of claims 1 to 3, further comprising a pull wire (340, 440, 540, 640) attached to the housing (302, 402, 502, 602) and extending proximally from the housing (302, 402, 502, 602) into a lumen of the catheter (206).

5. The bearing assembly of claim 4, wherein the pull wire (340, 440, 540, 640) is inserted into a pull-wire hole in the housing (302, 402, 502, 602).

6. The bearing assembly of claim 5, wherein the pull wire (340, 440) includes a retainer (342, 442) that prevents the pull wire (340, 440) from being pulled proximally through the pull-wire hole.

7. The bearing assembly of claim 4, wherein the housing (502, 602) includes a pull-wire groove (541, 641) and wherein the pull wire (540, 640) is inserted into, and attached to, the pull-wire groove (541, 641).

8. The bearing assembly of claim 4, wherein the housing (302, 602) includes a distal housing part (302d, 602d) joined to a proximal housing part (302p, 602p), and wherein the pull wire (340, 640) is coupled to the proximal housing part (302p, 602p).

9. The bearing assembly of claim 8, wherein the outer bearing race (318, 618) is formed in the distal housing part (302d, 602d).

10. The bearing assembly of any one of claims 1 to 9, further comprising an access hole (404, 504) extending through the housing (402, 502) from a radial wall (416) to an exterior of the housing (402, 502), wherein the plurality of ball bearings (428, 528) can be inserted into the bearing race through the access hole (404, 504).

11. The bearing assembly of claim 10, further comprising a stopper to seal the access hole (404, 504) after the plurality of ball bearings (428, 528) have been inserted.

12. The bearing assembly of any one of claims 1 to 11, wherein the docking cap stem (408, 508, 608) is integrated into a docking cap projection (408, 508, 608), so that the docking cap (406, 506, 606) and the docking cap stem (408, 508, 608) are a single piece.

13. A biostimulator transport system (202) comprising:
a catheter (206, 304) having a lumen, and a torque shaft (305) positioned in the lumen; and
the bearing assembly (300, 400, 500, 600) of any one of claims 1 to 12 coupled to the catheter (206, 304).

14. A biostimulator system (200) comprising:
the biostimulator transport system (202) of claim 13; and
a biostimulator (100) mounted to the docking cap (306, 406, 506, 606).

15. The biostimulator system of claim 14, wherein the biostimulator (100) is a leadless pacemaker.

## Patentansprüche

1. Lageranordnung (300, 400, 500, 600), umfassend:
ein Gehäuse (302, 402, 502, 602), das dazu ausgelegt ist, mit einem Katheter (206, 304) gekoppelt zu werden, wobei das Gehäuse (302, 402, 502, 602) eine zentrale Öffnung (320, 420, 520, 620) und einen äußeren Lagerring (318, 418, 518, 618) beinhaltet, der in dem Gehäuse (302, 402, 502, 602) um die zentrale Öffnung (320, 420, 520, 620) herum gebildet ist;
einen Andockkappenschaft (308, 408, 508, 608), der in der zentralen Öffnung (320, 420, 520, 620) positioniert und relativ zu dem Gehäuse (302, 402, 502, 602) drehbar ist, wobei der Andockkappenschaft (308, 408, 508, 608) dazu ausgelegt ist, mit einer Drehmomentwelle (305) des Katheters (206, 304) gekoppelt zu werden;
eine Mehrzahl von Kugellagern (328, 428, 528, 628); und
eine Andockkappe (306, 406, 506, 606), die mit dem Andockkappenschaft (308, 408, 508, 608) gekoppelt ist, **dadurch gekennzeichnet, dass**
der Andockkappenschaft (308, 408, 508, 608) einen inneren Lagerring (316, 426, 526, 626) bildet und wobei der äußere Lagerring (318, 418, 518, 618) und der innere Lagerring (316, 426, 526, 626) zusammen einen Lagerring bilden; und
die Mehrzahl von Kugellagern (328, 428, 528, 628) in dem Lagerring positioniert und in diesem enthalten ist.

2. Lageranordnung nach Anspruch 1, ferner umfassend eine Dichtung (336, 436, 536, 636), die zwischen der Andockkappe (306, 406, 506, 606) und dem Gehäuse (302, 402, 502, 602) positioniert ist.

3. Lageranordnung nach Anspruch 2, wobei die Dichtung (336, 436, 536, 636) ein O-Ring ist.

4. Lageranordnung nach einem der Ansprüche 1 bis 3, ferner umfassend einen Zugdraht (340, 440, 540, 640), der an dem Gehäuse (302, 402, 502, 602) befestigt ist und sich proximal von dem Gehäuse (302, 402, 502, 602) in ein Lumen des Katheters (206) erstreckt.

5. Lageranordnung nach Anspruch 4, wobei der Zugdraht (340, 440, 540, 640) in ein Zugdrahtloch in dem Gehäuse (302, 402, 502, 602) eingesetzt ist.

6. Lageranordnung nach Anspruch 5, wobei der Zugdraht (340, 440) eine Halterung (342, 442) beinhaltet, die verhindert, dass der Zugdraht (340, 440) proximal durch das Zugdrahtloch gezogen wird.

7. Lageranordnung nach Anspruch 4, wobei das Gehäuse (502, 602) eine Zugdrahtnut (541, 641) beinhaltet und wobei der Zugdraht (540, 640) in die Zugdrahtnut (541, 641) eingesetzt und daran angebracht ist.

8. Lageranordnung nach Anspruch 4, wobei das Gehäuse (302, 602) einen distalen Gehäuseteil (302d, 602d) beinhaltet, der mit einem proximalen Gehäuseteil (302p, 602p) verbunden ist, und wobei der Zugdraht (340, 640) mit dem proximalen Gehäuseteil (302p, 602p) gekoppelt ist.

9. Lageranordnung nach Anspruch 8, wobei der äußere Lagerring (318, 618) in dem distalen Gehäuseteil (302d, 602d) gebildet ist.

10. Lageranordnung nach einem der Ansprüche 1 bis 9, ferner umfassend ein Zugriffsloch (404, 504), das sich von einer radialen Wand (416) durch das Gehäuse (402, 502) zu einem Äußeren des Gehäuses (402, 502) erstreckt, wobei die Mehrzahl von Kugellagern (428, 528) durch das Zugriffsloch (404, 504) in den Laufring eingeführt werden kann.

11. Lageranordnung nach Anspruch 10, ferner umfassend einen Anschlag zum Abdichten des Zugangslochs (404, 504), nachdem die Mehrzahl von Kugellagern (428, 528) eingeführt wurde.

12. Lageranordnung nach einem der Ansprüche 1 bis 11, wobei der Andockkappenschaft (408, 508, 608) in einen Andockkappenvorsprung (408, 508, 608) integriert ist, sodass die Andockkappe (406, 506, 606) und der Andockkappenschaft (408, 508, 608) ein einziges Stück sind.

13. Biostimulatortransportsystem (202), umfassend:
einen Katheter (206, 304), der ein Lumen und eine Drehmomentwelle (305) aufweist, die in dem Lumen positioniert ist; und
die Lageranordnung (300, 400, 500, 600) nach einem der Ansprüche 1 bis 12, die mit dem Katheter (206, 304) gekoppelt ist.

14. Biostimulatorsystem (200), umfassend:
das Biostimulatortransportsystem (202) nach Anspruch 13; und
einen Biostimulator (100), der an der Andockkappe (306, 406, 506, 606) montiert ist.

15. Biostimulatorsystem nach Anspruch 14, wobei der Biostimulator (100) ein leitungsloser Schrittmacher ist.

## Revendications

1. Ensemble de palier (300, 400, 500, 600), comprenant :
un logement (302, 402, 502, 602) conçu pour le couplage à un cathéter (206, 304), le logement (302, 402, 502, 602) incluant une ouverture centrale (320, 420, 520, 620) et une bague de roulement externe (318, 418, 518, 618) formée dans le logement (302, 402, 502, 602) autour de l'ouverture centrale (320, 420, 520, 620) ;
une tige de capuchon d'amarrage (308, 408, 508, 608) positionnée dans l'ouverture centrale (320, 420, 520, 620) et apte à tourner par rapport au logement (302, 402, 502, 602), la tige de capuchon d'amarrage (308, 408, 508, 608) étant conçue pour le couplage à un arbre de torsion (305) du cathéter (206, 304) ;
une pluralité de roulements à billes (328, 428, 528, 628) ; et
un capuchon d'amarrage (306, 406, 506, 606) couplé à la tige de capuchon d'amarrage (308, 408, 508, 608), **caractérisé en ce que**
la tige de capuchon d'amarrage (308, 408, 508, 608) forme une bague de roulement interne (316, 426, 526, 626) et dans lequel la bague de roulement externe (318, 418, 518, 618) et la bague de roulement interne (316, 426, 526, 626) forment ensemble une bague de roulement ; et
la pluralité de roulements à billes (328, 428, 528, 628) sont positionnés dans la bague de roulement et contenus par celle-ci.

2. Ensemble de palier selon la revendication 1, comprenant en outre un joint (336, 436, 536, 636) positionné entre le capuchon d'amarrage (306, 406, 506, 606) et le logement (302, 402, 502, 602).

3. Ensemble de palier selon la revendication 2, dans lequel le joint (336, 436, 536, 636) est un joint torique.

4. Ensemble de palier selon l'une quelconque des revendications 1 à 3, comprenant en outre un fil de traction (340, 440, 540, 640) attaché au logement (302, 402, 502, 602) et s'étendant de manière proximale à partir du logement (302, 402, 502, 602) dans une lumière du cathéter (206).

5. Ensemble de palier selon la revendication 4, dans lequel le fil de traction (340, 440, 540, 640) est inséré dans un trou de fil de traction dans le logement (302, 402, 502, 602).

6. Ensemble de palier selon la revendication 5, dans lequel le fil de traction (340, 440) inclut un dispositif de retenue (342, 442) qui empêche le fil de traction (340, 440) d'être tiré de manière proximale à travers le trou de fil de traction.

7. Ensemble de palier selon la revendication 4, dans lequel le logement (502, 602) inclut une rainure de fil de traction (541, 641) et dans lequel le fil de traction (540, 640) est inséré dans la rainure de fil de traction (541, 641) et attaché à celle-ci.

8. Ensemble de palier selon la revendication 4, dans lequel le logement (302, 602) inclut une partie de logement distale (302d, 602d) jointe à une partie de logement proximale (302p, 602p), et dans lequel le fil de traction (340, 640) est couplé à la partie de logement proximale (302p, 602p).

9. Ensemble de palier selon la revendication 8, dans lequel la bague de roulement externe (318, 618) est formée dans la partie de logement distale (302d, 602d).

10. Ensemble de palier selon l'une quelconque des revendications 1 à 9, comprenant en outre un trou d'accès (404, 504) s'étendant à travers le logement (402, 502) depuis une paroi radiale (416) jusqu'à un extérieur du logement (402, 502), dans lequel la pluralité de roulements à billes (428, 528) peuvent être insérés dans la bague de roulement à travers le trou d'accès (404, 504).

11. Ensemble de palier selon la revendication 10, comprenant en outre un bouchon pour sceller le trou d'accès (404, 504) après que la pluralité de roulements à billes (428, 528) ont été insérés.

12. Ensemble de palier selon l'une quelconque des revendications 1 à 11, dans lequel la tige de capuchon d'amarrage (408, 508, 608) est intégrée dans une saillie de capuchon d'amarrage (408, 508, 608), de sorte que le capuchon d'amarrage (406, 506, 606) et la tige de capuchon d'amarrage (408, 508, 608) sont une pièce unique.

13. Système de transport de biostimulateur (202) comprenant :
un cathéter (206, 304) ayant une lumière, et un arbre de torsion (305) positionné dans la lumière ; et
l'ensemble de palier (300, 400, 500, 600) selon l'une quelconque des revendications 1 à 12 couplé au cathéter (206, 304).

14. Système de biostimulateur (200) comprenant :
le système de transport de biostimulateur (202) selon la revendication 13 ; et
un biostimulateur (100) monté sur le capuchon d'amarrage (306, 406, 506, 606).

15. Système de biostimulateur selon la revendication 14, dans lequel le biostimulateur (100) est un stimulateur cardiaque sans fil.
